(19) Europäisches Patentamt
European Patent Office
Office européen des brevets

(11) Publication number: **0 002 021**
**A1**

# (12) EUROPEAN PATENT APPLICATION

(21) Application number: **78101320.6**

(22) Date of filing: **07.11.78**

(51) Int. Cl.²: **C 07 C 17/34**, C 07 C 21/06

(30) Priority: **18.11.77 US 852888**

(43) Date of publication of application: **30.05.79 Bulletin 79/11**

(84) Designated Contracting States: **BE DE FR GB NL SE**

(71) Applicant: **The B.F. GOODRICH Company, Dept. 0015 WHB-6 500 South Main Street, Akron, Ohio 44318 (US)**

(72) Inventor: **Magistro, Angelo Joseph, 11017 Brookview Drive, Brecksville Ohio 44141 (US)**

(74) Representative: **Baillie, Iain Cameron et al, c/o Ladas, Parry, von Gehr. Goldsmith & Deschamps Blumenstrasse 48, D-8000 München 2 (DE)**

## (54) Catalytic dehydrohalogenation process.

(57) There is presented an improved process for making vinyl chloride from ethylene dichloride. Specifically, the improvement comprises cracking ethylene dichloride in the presence of a zeolite which has been treated or reacted with a Lewis acid. This enables one to conduct the dehydrohalogenation or cracking operation at lower temperatures than usually employed with increased conversion of ethylene dichloride to vinyl chloride.

EP 0 002 021 A1

# CATALYTIC DEHYDROHALOGENATION PROCESS

## BACKGROUND OF THE INVENTION

It is well known that vinyl chloride can be produced from ethylene dichloride (EDC) by means of a cracking process or pyrolysis by splitting off a molecule of hydrogen chloride. The vinyl chloride is obtained in the form of vapor and then condensed. The cracking is accomplished in the absence of a catalyst by heating the EDC in an inert atmosphere under high temperature and pressure. Usually a temperature in the range of about 500 C. to about 600 C. and a pressure of about 500 to 600 psig is used. The generation of such energy, of course, is espensive.

Generally, in the process of making vinyl chloride by the dehydrohalogenation of EDC, in the absence of a catalyst, the reaction is conducted in tubes having a restricted cross-sectional area. One serious difficulty encountered in such a process is the relatively rapid deposition of solid or high-boiling by-products which cokeup the tubes and limit the process to the utilization of relatively short operating periods. The deposits which are fromed are generally of such a nature that it requires frequent opening of the tubes and removal of the deposit therefrom. This results in a significant amount of down time with the resultant loss of production.

When a catalyst is not employed, you have undesirable side reactions and low order of conversion and yields. Various catalytic processes have been proposed which processes are generally characterized by the particular

catalytic material employed. While a number of such catalytic processes have had commercial success, there has been and still is, a continuing search for catalytic materials which will improve the dehydrohalogenation or cracking process by further reducing undesirable side reactions and increasing conversion.

Another aspect of the dehydrohalogenation or cracking processes is the high temperatures that are necessary, whether or not they are noncatalytic or catalytic. This requires expending a great deal of energy, which in many cases makes the processes impractical because of the economics. Accordingly, it would be most desirable to have a process which could be operated at low temperatures, that is, much lower than at present thereby conserving energy without affecting conversion and hopefully with increased conversion. At the present time, with the Government's emphasis on the conservation of energy, this becomes most important.

## SUMMARY OF THE INVENTION

I have now unexpectedly found that improved conversion with less energy can be obtained by a process which comprises cracking or dehydrohalogenating ethylene dichloride (EDC) to vinyl chloride by employing, as catalyst, various zeolites which have been treated or reacted with Lewis acids. The use of the so treated zeolite cracking catalysts enables operation at temperatures far below that normally required with prior processes. Using the catalysts of the instant

invention, the cracking process can be operated at a temperature in the range of about 200°C. to about 400°C.

## DETAILED DESCRIPTION

Vinyl chloride is prepared by various processes from ethylene, elemental chlorine, and/or hydrogen chloride in most all of which a cracking or dehydrohalogenation step is employed wherein ethylene dichloride is thermally cracked in the vapor phase under pressure to vinyl chloride and by-product hydrogen chloride. The latter is recovered by an oxyhydrochlorination step wherein the hydrogen chloride is reacted with additional ethylene and oxygen to produce dichloroethanes which in turn are recycled to the cracking step. In many processes, a direct chlorination step is also employed wherein ethylene and elemental chlorine are reacted in liquid phase to produce dichloroethanes which are then cracked to vinyl chloride.

In the overall process for making vinyl chloride the first step is the vapor phase hot chlorination of ethylene to produce EDC. This reaction is strongly exothermic and liberates its heat at temperatures sufficiently high to crack EDC. On the other hand, the cracking of EDC is an endothermic reaction. Theoretically the two reactions should supply a small excess of heat but due to various heat losses and the heat required to preheat feed streams, it is necessary to maintain a net heat input into the process.

The oxyhydrochlorination reaction or step is

strongly exothermic and, as commonly carried out in a fluidized bed reactor, is a significant net low pressure steam generator. The EDC produced in the oxyhydrochlorination reaction is fed to the cracking step or cracker where it is cracked to vinyl chloride. The heat absorbed in cracking such recycled EDC has to be supplied to the cracker and such heat load, plus the heat energy requirements of the vinyl chloride recovery and purification train, are the main heat requirements of the overall process. In many prior art vinyl chloride processes, large amounts of natural gas are required to operate the known forms of the direct fired tube-type cracking furnaces and gas-fired burner type by-product disposal furnaces.

In prior processes, the EDC, which is a mixture comprised essentially of 1,2-dichloroethane and some 1,1-dichloroethane, is vaporized and the vapors passed through a tube-type cracking furnace which usually is directly heated by gas burners to a temperature in the range of about 450°C. to about 650°C. Usually no cracking catalyst is employed but there are prior art processes which disclose the use of cracking tubes packed with either porous inert solids or with other type catalysts which are alleged to facilitate the reaction. In the absence of a catalyst, the reaction is a thermally induced cracking whereby one molecule of hydrogen chloride is split out of each EDC molecule forming one molecule of vinyl chloride.

In the cracking reaction, pressures will range

anywhere from 1 atmosphere, or slightly below, up to 28 to 48 atmospheres. The mixed vapors from the cracking reaction are cooled to condense the bulk of the chlorohydrocarbons. The recovered chlorohydrocarbons are then fractionated in one or more columns to separate hydrogen chloride, vinyl chloride, unreacted EDC and impurities. In this fractionation, low and high boiling impurities, or by-products, are also separated out which are then fed to a catalytic combustion reactor where they are converted to primarily hydrogen chloride. The by-product HCl from the cracker and the HCl from the combustion reactor are recycled to the oxyhydrochlorination step to produce additional EDC which, along with the recovered unreacted EDC, is returned to the cracking step.

The subject of the present invention comprises a new and improved cracking step for use in the manufacture of vinyl chloride. Specifically, the invention comprises cracking EDC in the presence of a catalyst, which catalyst is a zeolite which has been treated or reacted with a volatile Lewis acid. For example, a zeolite such as faujasite Y reacted with the Lewis acid $TiCl_4$. Such a reaction is represented by the following equation:

$$\equiv SiOH + TiCl_4 \longrightarrow \equiv Si\text{-}O\text{-}TiCl_3 + HCl \ .$$

Both natural and synthetic zeolites may be used in the present invention. The zeolites are also known as molecular sieves. The natural zeolites are analcite, chabazite, heulandite, natrolite, stilbite, and thomsonite

and are classified as minerals. The synthetic or artificial zeolites can be made in a variety of forms ranging from gelatinous to porous and sandlike. For the purposes of the present invention, the solid zeolites are preferred. The most preferred synthetic zeolites for use in the present invention are the faujasite Y zeolites since they are readily available commercially. These zeolites are obtainable from the Linde Division of Union Carbide Corporation of New York, New York, and are sold as "Linde Molecular Sieves" with a code number. As examples of such zeolites may be mentioned Na-Y, Ca-Y, H-Y, etc. A good reference describing these zeolites is found in Adv. in Catalysis, Vol. 18 (1968), pages 277-371.

With respect to the Lewis acid, a great number may be used in the present invention. Preferably, a Lewis acid containing chlorine is employed since the invention is concerned with making vinyl chloride. In "The Condensed Chemical Dictionary", eighth edition, published by Van Nostrand Reinhold Company, Lewis acid is defined as follows: "Any molecule or ion that can combine with another molecule or ion by forming a covalent chemical bond with two electrons from the second molecule or ion. An acid is thus an electron acceptor. Hydrogen ion, or the proton, is the simplest substance that will do this, but many other substances, such as boron trifluoride, $BF_3$, and aluminum chloride, $AlCl_3$, exhibit the same behavior and are therefore properly called acids. Such substances show acid effects on indicator colors

and when dissolved in the proper solvents." As examples of the Lewis acids useful in the present invention there may be named $TiCl_4$, $SbCl_3$, $SbCl_5$, $AlCl_3$, $PCl_3$, $SnCl_4$, $ZnCl_2$, and the like.

The zeolite chosen should be a solid since it will be packed in the cracking reactor. The treatment or reaction of the zeolite with the Lewis acid can be done in a separate reactor prior to placing the so produced catalyst in the cracking reactor. However, for convenience of handling, the zeolite is placed in the cracking reactor prior to reaction with the Lewis acid. Nitrogen is then introduced into the reactor and the reactor and zeolite is heated to about 300°C. At the bottom of the reactor below the zeolite the temperature is 100°C. The Lewis acid, for example $TiCl_4$, is injected into the nitrogen stream entering the reactor where it is vaporized and carried into contact with the zeolite and the reaction takes place with by-product hydrogen chloride being taken off at the top of the reactor or cracker. The amount of HCl is measured by simple titration with a base in order to determine the extent of the reaction. A reaction of about 5.0% to about 100.0% of the surface is capable to prepare the catalyst having the desirable activity. However, the amount of reaction can be less than 5% and still have an active catalyst.

It is to be understood that the reacted zeolite catalyst is placed in the cracking reactor in such fashion as to allow the rapid passage of vapor or gas therethrough. The

catalyst in the reactor may be either a fixed bed or a fluidized bed. Preferably, the cracking step is done in an inert atmosphere, nitrogen being particularly good for this purpose. After the cracking reactor has been purged with nitrogen, the EDC, in gaseous form, is introduced into the reactor in the nitrogen stream. When the EDC comes in contact with the catalyst the dehydrochlorination reaction or cracking proceeds smoothly and rapidly converting the EDC to vinyl chloride and by-product hydrogen chloride. As previously pointed out, the temperature in the cracker is maintained in the range of about 200°C. to about 400°C. Temperatures lower than 200°C. may be employed or one may use temperatures higher than 400°C. However, optimum results are obtained when operating within the temperature range given above.

While the cracking reaction may be operated at atmospheric pressure, or slightly below, it is preferred in the present invention to operate at superatmospheric pressure. A pressure anywhere up to 28 to 48 atmospheres is satisfactory. At higher pressures there is less cracking of the EDC into undesirable chlorohydrocarbon by-products, such as $CCl_4$, etc., than when operating at atmospheric pressure. Further, when using superatmospheric pressure, less coking or carbon formation occurs. Periodically the reactor is shut down and the carbon or coke formation, if any, is removed, usually by burning off, that is, heating the reactor at a high temperature. Usually a temperature in the

range of about 300°C. to about 700°C. is sufficient to remove the coke formation.

The reaction or contact time of the EDC vapor with the catalyst in the reactor can be varied. The contact time necessary between the EDC and catalyst to promote the desired dehydrochlorination reaction is obtained by controlling the space velocity of the gaseous material passing through the reaction zone. The contact time is dependent upon several factors, namely, the scale of the operation, the quantity of catalyst in the reactor or cracker, and the type of reactor employed. It has been found, however, that for most reactors a contact time as high as about 25 seconds or more and as low as 0.5 second can be employed. If the contact time is too low the quantity of unreacted EDC coming over is too high. On the other hand, if the contact time is too high, that is, much above 25 seconds, the impurities increase which makes it more difficult to recover vinyl chloride in a pure form. One can readily adjust the gaseous feed rate to obtain the optimum reaction or contact time for any particular type reactor.

The gaseous mixture that is withdrawn from the cracker or reaction zone can be passed directly to a condenser thus recovering the condensable materials and allowing the hydrogen chloride to pass overhead and recycling the same to the oxyhydrochlorination step or zone to be used in making more EDC. Alternatively, the gases leaving the reaction zone can be cooled and subjected to fractional distillation under

superatmospheric pressure, preferably at the same or lower pressure as that used for the cracking. The hydrogen chloride separates first and is recycled, as above indicated. The uncondensed gases are removed by releasing the pressure. These gases, which consist mainly of vinyl chloride and unreacted EDC are condensed and fractionally distilled to remove the vinyl chloride. The unreacted EDC recovered is recycled to the cracking or reaction zone.

The following examples are given to more specifically define the instant invention. It is understood that this is intended in an illustrative and not limitative sense. In the examples all parts and percents are by weight, unless otherwise indicated.

EXAMPLE I

In this Example, two catalysts were prepared in accordance with the invention and unreacted zeolite was run for comparison purposes. First, 20 cc. of zeolite H-Y (11.0 gms.) were heated at 300°C. for one hour with nitrogen. Then 4.10 grams $TiCl_4$ were added at 300°C. with nitrogen in about 8 hours. This was allowed to run overnight with nitrogen at 300°C. Using the same procedure, a second catalyst was made using 26 cc. of zeolite Ca-Y and heating the same with nitrogen for 1 hour at 300°C. Then 4.36 gms. of $SbCl_5$ was vaporized onto the Ca-Y under nitrogen at 300°C. This was then kept at 300°C. overnight under nitrogen. The catalysts were placed in vertical tube reactors and nitrogen and EDC in gaseous form was run through the reactors in contact with

the catalysts, using zeolite alone as the control. The conditions of reaction and the results are shown in the following table:

TABLE I

| Run No. | Zeolite | Lewis Acid | Cracking Temp. °C. | Contact Time Seconds | EDC Conversion % | Ratio $N_2$/EDC |
|---|---|---|---|---|---|---|
| 1 | H-Y | None | 251 | 6.0 | 18.0 | 6/1 |
| 2 | H-Y | $TiCl_4$ | 250 | 5.27 | 32.6 | 5/1 |
| 3 | Ca-Y | None | 230 | 6.68 | 28.9 | 6/1 |
| 4 | Ca-Y | $SbCl_5$ | 225 | 6.84 | 50.1 | 5/1 |

It can readily be seen from the Table I that the conversion of EDC to vinyl chloride is almost double when using the catalysts of the present invention. Also, the cracking temperature is about half that required in prior art processes irrespective of whether a catalyst or no catalyst is employed.

EXAMPLE II

In this Example a number of runs were made using a vertical tube reactor or cracker and running nitrogen and EDC in gaseous form over a number of different catalysts. The catalysts were prepared as outlined in Example I using various zeolites and Lewis acids. In these runs the yield of vinyl chloride was determined in addition to the percent conversion of EDC to vinyl chloride. In these runs, the contact time and cracking temperature was varied. The results are tabulated in the following table:

TABLE II

| Run No. | Catalyst | Mole Ratio $N_2$/EDC | Cracking Temp. °C. | Mole % Conversion EDC | % Yield Vinyl Chloride | Carbon Balance | Contact Time Seconds |
|---|---|---|---|---|---|---|---|
| 5 | Ca-Y+$TiCl_4$ | 6/1 | 225 | 32.7 | 100.0 | 93.4 | 6.3 |
| 6 | Ca-Y+$TiCl_4$ | 6/1 | 250 | 32.9 | 97.6 | 93.8 | 6.2 |
| 7 | Ca-Y+$TiCl_4$ | 6/1 | 275 | 49.4 | 96.3 | 97.0 | 6.0 |
| 8 | Ca-Y+$SbCl_5$ | 5/1 | 225 | 50.2 | 92.6 | 63.8 | 6.8 |
| 9 | Ca-Y+$SiCl_4$ | 5/1 | 225 | 20.5 | 100.0 | 99.5 | 6.8 |
| 10 | Ca-Y+$PCl_3$ | 5/1 | 225 | 16.0 | 100.0 | 94.3 | 6.8 |
| 11 | H-Y+$TiCl_4$ | 5/1 | 250 | 32.6 | 100.0 | 90.3 | 5.3 |
| 12 | NA-Y+$SiCl_4$ | 5/1 | 350 | 46.8 | 100.0 | 88.4 | 4.0 |
| 13 | Na-Y+$TiCl_4$ | 5/1 | 250 | 16.9 | 100.0 | 85.4 | 4.8 |
| 14 | Na-Y+$TiCl_4$ | 5/1 | 300 | 20.3 | 78.6 | 92.9 | 4.5 |

Table II shows the varying catalysts and the good yields of vinyl chloride that are obtained with the new catalysts of the present invention.

As has been previously pointed out, the improved EDC cracking process of the instant invention has the advantage of allowing one to operate at much lower temperatures than prior art processes with increased conversion of EDC to vinyl chloride. Numerous other advantages of the invention will be apparent to those skilled in the art.

While the present invention has been described in terms of its specific embodiments, certain modifications and equivalents will be apparent to those skilled in the art and are intended to be included within the scope of the invention, which is to be limited only by the scope of the appended claims.

0002021

CLAIMS

I CLAIM:

1. A process for making vinyl chloride which comprises passing a gaseous stream containing ethylene dichloride in contact with a catalyst in a heated reaction zone and recovering said vinyl chloride from the reaction products, said catalyst being the reaction product of a zeolite and a Lewis acid.

2. A process as defined in Claim 1 wherein the reaction zone is maintained at a temperature in the range of from about 200°C. to about 400°C.

3. A process as defined in Claim 1 wherein the zeolite is a faujasite Y.

4. A process as defined in Claim 1 wherein the ethylene dichloride is in contact with the catalyst for a period of time from about 0.5 second to about 25 seconds.

5. A process as defined in Claim 1 wherein the Lewis acid is $TiCl_4$.

6. A process as defined in Claim 1 wherein the Lewis acid is $SbCl_5$.

7. A process as defined in Claim 1 wherein the Lewis acid is $PCl_3$.

8. A process as defined in Claim 1 wherein the pressure in the reaction zone is in the range of less than atmospheric up to about 48 atmospheres.

9. A process as defined in Claim 2 wherein the zeolite is Ca-Y and the Lewis acid is $TiCl_4$.

10. A process as defined in Claim 2 wherein the zeolite is H-Y and the Lewis acid is $TiCl_4$.

11. A process as defined in Claim 2 wherein the zeolite is Na-Y and the Lewis acid is $TiCl_4$.

12. A process as defined in Claim 3 wherein the reaction zone is maintained at a temperature in the range of from about 200°C. to about 400°C.

13. A process as defined in Claim 12 wherein the ethylene dichloride is in contact with the catalyst for a period of time from about 0.5 second to about 25 seconds.

European Patent Office

# EUROPEAN SEARCH REPORT

Application number
EP 78 10 1320

## DOCUMENTS CONSIDERED TO BE RELEVANT

| Category | Citation of document with indication, where appropriate, of relevant passages | Relevant to claim |
|---|---|---|
| D | ADVANCES IN CATALYSIS, vol. 18, 1968, Academic Press, New York, London, pages 311-313<br>* Pages 311-313 *<br>-- | 1-13 |
| | ZEOLITE CHEMISTRY AND CATALYSIS, 1976, Jule A. Rabo, Editor, American Chemical Society, Washington DC, USA, pages 556,557, 587-588.<br>* Page 556 under to page 577 top page 587 "other reactions"; page 588, table XII *<br>-- | 1-13 |
| | US - A - 3 927 131 (J.A. WARD)<br>* Columns 5,6; claims 1,2,4; columns 3,4; examples 4,5 *<br>-- | 1-13 |
| | GB - A - 1 152 021 (PULLMAN INC.)<br>* Pages 5,6,7; examples 1-7 *<br>-- | 1-13 |
| | CHEMICAL ABSTRACTS, vol. 86, 15989d (1977), page 368. Colombus, Ohio, USA KLADNIG et al. "Dehydrochlorination of 1- and 2-chlorobutane over 13-X and 4-A zeolites with different cations".<br>& Acta Cient. Venez.,Supl. 1973, 24(2), 142-145.<br>* Abstract *<br>---- | 1-13 |

CLASSIFICATION OF THE APPLICATION (Int. Cl.²)

C 07 C 17/34
21/06

TECHNICAL FIELDS SEARCHED (Int.Cl.²)

C 07 C 17/34
21/06

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 18-01-1979 | BESLIER |